(19) **Europäisches Patentamt**

**European Patent Office**

**Office européen des brevets**

(11) Publication number: **0 260 859**
**A1**

## (12) EUROPEAN PATENT APPLICATION

(21) Application number: **87307907.3**

(22) Date of filing: **08.09.87**

(51) Int. Cl.4: **A61L 25/00** , **A61K 9/70**

---

Claims for the following Contracting States: AT + ES + GR.

(30) Priority: **19.09.86 US 909141**

(43) Date of publication of application:
**23.03.88 Bulletin 88/12**

(84) Designated Contracting States:
**AT BE CH DE ES FR GB GR IT LI LU NL SE**

(71) Applicant: **PFIZER INC.**
**235 East 42nd Street**
**New York, N.Y. 10017(US)**

(72) Inventor: **Scheps, Milton Harold**
**11203 Hammock Drive**
**Largo Florida(US)**

(74) Representative: **Wood, David John et al**
**Pfizer Limited Ramsgate Road**
**Sandwich Kent CT13 9NJ(GB)**

---

(54) **A medicated skin preparation.**

(57) A medicated skin composition comprises a mixture of a copolymer of methylvinylether and methacrylic acid, isopropyl alcohol, citric acid ester plasticizer and 2,4,4'-trichloro-2'-hydroxydiphenyl ether. This composition is applied to the skin to form an antimicrobial barrier.

EP 0 260 859 A1

# A MEDICATED SKIN PREPARATION

This invention relates to a medicated skin composition and a process for forming an antimicrobial barrier on skin by applying said medicated skin composition to the skin.

Medicated skin compositions are known and are currently on the market. One commercial product at the moment is Sween Prep (trade mark) which comprises chloroxylenol as a bacteriostat. Although some antimicrobial activity is attained with such commercial products, the duration of activity has been limited.

The medicated skin composition having extended antimicrobial activity according to the invention comprises a film-forming amount of a copolymer of methylvinylether and methacrylic acid, isopropyl alcohol solvent, a plasticizing amount of citric acid ester plasticizer and an antimicrobially effective amount of 2,4,4'-trichloro-2'-hydroxydiphenyl ether.

Said composition is applied to the skin to form an antimicrobial barrier on the skin.

The film-forming copolymer of methylvinylether and maleic acid is present in the composition in sufficient amount to form a film on the skin on application to the skin. Such amount generally ranges from about 10 to 20% by weight of the total composition. The copolymer is usually present in the form of its $C_1$-$C_6$-alkyl ester, e.g. the butyl ester. A useful product is Gantrez® ES-435 of GAF Corporation which is the butyl monoester of poly-(methyl vinyl ether and maleic acid).

The isopropyl alcohol is present in an amount sufficient to dissolve the other ingredients of the composition. Generally, the alcohol solvent will be present in an amount ranging from about 80 to 90% by weight of the total composition.

The citric acid ester plasticizer is present in a plasticizing amount generally ranging from about 0.1 to 3% by weight of the total composition. Examples of suitable citric acid ester plasticizers are trialkyl esters of citric acid wherein each alkyl has from 1 to 6 carbon atoms. A preferred citric acid ester is acetyl tributyl citrate, which is available as Citroflex® A-4.

The antimicrobial compound 2,4,4'-trichloro-2'-hydroxydiphenyl ether (Triclosan) is present in an amount effective for antimicrobial activity of the skin composition, generally in an amount from 0.1 to 2% by weight of the total composition.

The skin composition of the invention is prepared by thorough mixing of the ingredients according to procedures standard in the preparation of medicated skin compositions. Tanks, homogenizer and other manufacturing equipment coming into contact with the ingredients of the skin composition are generally washed with hot detergent solution, rinsed, sanitized with an antiseptic such as a chlorine solution and again rinsed before use.

The skin composition may be made in different forms, e.g. as a brush-on liquid, an aerosol spray, a medicated skin wipe or a swab.

The brush-on liquid is generally prepared by slowly adding 2,4,4'-trichloro-2'-hydroxydiphenyl ether to isopropylalcohol solvent, and thorough mixing until dissolution of the ether in the solvent. The citric acid ester plasticizer and the copolymer of methylvinylether and maleic acid are then added and mixed until dissolution and formation of a homogeneous solution.

The aerosol spray is generally prepared by mixing the medicated skin composition of the invertion with a conventional propellant such as hydrocarbons, e.g. propane or butane, and fluorinated hydrocarbons. Commonly, equal weight amounts of the medicated skin composition and the propellant are used.

The medicated skin wipe may be prepared by impregnating a paper web material with a homogeneous solution such as described above for use as a brush-on liquid. The medicated skin wipe is packaged in an air-tight enclosure to avoid unsanitary conditions before use. The package material is conveniently a three ply foil laminate consisting of a paper layer, a polypropylene layer and a layer of Surlyn®, which is a thermoplastic polymer.

The following examples illustrate the invention.

## Example 1

The following ingredients are combined in the percentage by weight indicated:

| Medicated Skin Preparation | |
|---|---|
| Acetyl tributyl citrate | 0.42% b.w. |
| Triclosan | 0.25% b.w. |
| Gantrez ES-435 | 14.00% b.w. |
| Isopropyl alcohol | 85.33% b.w. |

Isopropyl alcohol is added to a presanitized, stainless steel vessel equipped with turbine agitation. Triclosan is slowly added and mixed well until complete dissolution. The acetyl tributyl citrate and Gantrez ES-435 are added and mixed thoroughly for complete dissolution and obtaining a homogeneous solution.

## Example 2

### Aerosol

The following ingredients are combined in the percentage by weight indicated: Medicated Skin Preparation of Example 1 except for isopropyl alcohol 24.0% b.w.
Isopropyl alcohol)(99%)    26.0% b.w.
Propellant A-46 [(1)]    50.0% b.w.

The medicated skin preparation and the isopropyl alcohol are mixed with a high speed mixer for 15 minutes. The mixture is filled into aerosol cans with an aerosol filling machine. The propellant is added to each can with the filling machine. Each can contains 4 3/8 oz.wt. of product of which 2 3/16 oz.wt. is the medicated skin preparation and 2 3/16 oz.wt. is the propellant.

## Claims

1. A medicated skin composition which comprises a mixture of a film-forming amount of a copolymer of methyl. vinyl ether and maleic acid, isopropyl alcohol solvent, a plasticizing amount of a citric acid ester plasticizer and an antimicrobially effective amount of 2,4,4'-trichloro-2'-hydroxydiphenyl ether.

2. A composition according to claim 1, characterized in that said plasticizer is acetyl tributyl citrate.

3. A composition according to claim 1 or 2, characterized in that said copolymer is present in an amount ranging from 10 to 20% by weight of the total composition.

4. A composition according to any one of claims 1 to 3,characterized in that said isopropyl alcohol solvent is present in an amount ranging from ,80 to 90% by weight of the total composition.

5. A composition according to any one of claims 1 to 4, characterized in that said plasticizer is present in an amount ranging from 0.1 to 3% by weight of the total composition.

6. A composition according to any one of claims 1 to 5, characterized in that said 2,4,4'-trichloro-2'-hydroxydiphenyl ether is present in an amount ranging from 0.1 to 2% by weight of the total composition.

7. A medicated skin composition according to claim 1, which is a mixture of about 85.3% isopropyl alcohol, about 0.4% acetyl tributyl citric acid, about 14% of methyl vinyl ether/maleic acid copolymer and about 0.3% of 2,4,4'-trichloro-2'-hydroxydiphenyl ether.

8. A process for forming an antimicrobial barrier on skin characterized by applying to the skin a medicated skin composition as claimed in any one of the preceding claims.

## Claims for the following Contracting State : AT

1. A process for preparing a medicated skin composition, characterized by combining a film-forming amount of a copolymer of methyl vinyl ether and maleic acid, isopropyl alcohol solvent, a plasticizing amount of a citric acid ester plasticizer and an antimicrobially effective amount of 2,4,4'-trichloro-2'-hydroxydiphenyl ether.

2. A process according to claim 1, characterized in that said plasticizer is acetyl tributyl citrate.

3. A process according to claim 1 or 2, characterized in that said copolymer is present in an amount ranging from 10 to 20% by weight of the total composition.

4. A process according to any one of claims 1 to 3, characterized in that said isopropyl alcohol solvent is present in an amount ranging from 80 to 90% by weight of the total composition.

5. A process according to any one of claims 1 to 4, characterized in that said plasticizer is present in an amount ranging from 0.1 to 3% by weight of the total composition.

6. A process according to any one of claims 1 to 5, characterized in that said 2,4,4'-trichloro-2'-hydroxydiphenyl ether is present in an amount ranging from 0.1 to 2% by weight of the total composition.

7. A process according to claim 1, characterized by combining about 85.3% isopropyl alcohol, about 0.4% acetyl tributyl citric acid, about 14% of methyl vinyl ether/maleic acid copolymer and about 0.3% of 2,4,4'-trichloro-2'-hydroxydiphenyl ether.

8. A process according to any one of the preceding claims, characterised in that the composition is prepared by the consecutive steps of (a) slowly adding the 2,4,4'-trichloro-2'-hydroxydiphenyl ether to the isopropyl alcohol solvent followed by thorough mixing until dissolution of the

[(1)]Mixture of propane and butane under a pressure of 46 pounds at 70°F.

ether in the solvent, (b) adding the citric acid ester plasticizer and the copolymer, and (c) mixing the ingredients until dissolution and formation of a homogeneous solution.

9. A process according to any one of the preceding claims, characterised by the further step of either mixing the composition with a propellant so as to form a composition suitable for an aerosol spray, or impregnating a paper web material with the composition so as to form a medicated skin wipe.

10. A medicated skin composition which comprises a mixture of a film-forming amount of a copolymer of methyl vinyl ether and maleic acid, isopropyl alcohol solvent, a plasticizing amount of a citric acid ester plasticizer and an antimicrobially effective amount of 2,4,4'-trichloro-2'-hydroxydiphenyl ether.

11. A process for forming an antimicrobial barrier on skin, characterized by applying to the skin a medicated skin composition as claimed in claim 10.

Claims for the following Contracting States : ES and GR

1. A process for preparing a medicated skin composition, characterized by combining a film-forming amount of a copolymer of methyl vinyl ether and maleic acid, isopropyl alcohol solvent, a plasticizing amount of a citric acid ester plasticizer and an antimicrobially effective amount of 2,4,4'-trichloro-2'-hydroxydiphenyl ether.

2. A process according to claim 1, characterized in that said plasticizer is acetyl tributyl citrate.

3. A process according to claim 1 or 2, characterized in that said copolymer is present in an amount ranging from 10 to 20% by weight of the total composition.

4. A process according to any one of claims 1 to 3,characterized in that said isopropyl alcohol solvent is present in an amount ranging from 80 to 90% by weight of the total composition.

5. A process according to any one of claims 1 to 4, characterized in that said plasticizer is present in an amount ranging from 0.1 to 3% by weight of the total composition.

6. A process according to any one of claims 1 to 5, characterized in that said 2,4,4'-trichloro-2'-hydroxydiphenyl ether is present in an amount ranging from 0.1 to 2% by weight of the total composition.

7. A process according to claim 1, characterized by combining about 85.3% isopropyl alcohol, about 0.4% acetyl tributyl citric acid, about 14% of methyl vinyl ether/maleic acid copolymer and about 0.3% of 2,4,4'-trichloro-2'-hydroxydiphenyl ether.

8. A process according to any one of the preceding claims, characterised in that the composition is prepared by the consecutive steps of (a) slowly adding the 2,4,4'-trichloro-2'-hydroxydiphenyl ether to the isopropyl alcohol solvent followed by thorough mixing until dissolution of the ether in the solvent, (b) adding the citric acid ester plasticizer and the copolymer, and (c) mixing the ingredients until dissolution and formation of a homogeneous solution.

9. A process according to any one of the preceding claims, characterised by the further step of either mixing the composition with a propellant so as to form a composition suitable for an aerosol spray, or impregnating a paper web material with the composition so as to form a medicated skin wipe.

10. A process for forming an antimicrobial barrier on skin, characterized by applying to the skin a medicated skin composition which has been prepared by a process as claimed in any one of claims 1 to 8.

## European Patent Office

### PARTIAL EUROPEAN SEARCH REPORT
which under Rule 45 of the European Patent Convention shall be considered, for the purposes of subsequent proceedings, as the European search report

Application number

EP 87 30 7907

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | JOURNAL OF PHARMACEUTICAL SCIENCES, vol. 63, no. 9, December 1974, pages 1376-1379 M. LUONGO et al.: "In vivo method for determining effectiveness of spray-on bandages containing anti-infectives" <br><br> * Page 1376, table 1 * | 1-7 | A 61 L 25/00 <br> A 61 K 9/70 |
| Y | FR - A - 2 397 186 (MALLINCKRODT INC) <br><br> * Page 7, line 34 - page 8, line 34; claims 1-9 * | 1-7 | |
| Y | AU - B - 527 826 (VITAPHARM PHARMACEUTICAL PROPRIETARY LTD) <br><br> * Page 2, line 8 - page 4, line 13 * | 1-7 | |
| A | EP - A - 0 048 556 (E.R. SQUIBB & SONS LTD) <br><br> * Claims 1-10 * | | **TECHNICAL FIELDS SEARCHED (Int. Cl.4)** <br><br> A 61 K <br> A 61 L |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-7

Claims searched incompletely:

Claims not searched: 8

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 08-12-1987 | TZSCHOPPE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82